(19) Europäisches Patentamt
European Patent Office
Office européen des brevets

(11) Veröffentlichungsnummer: **0 351 647 B1**

(12) EUROPÄISCHE PATENTSCHRIFT

(45) Veröffentlichungstag der Patentschrift: **30.12.92**

(21) Anmeldenummer: **89112304.4**

(22) Anmeldetag: **06.07.89**

Die Akte enthält technische Angaben, die nach dem Eingang der Anmeldung eingereicht wurden und die nicht in dieser Patentschrift enthalten sind.

(51) Int. Cl.5: **C07C 61/15**, C07C 47/353, C07C 31/44, C07C 49/327, C07C 69/635, C07C 265/10, C07C 233/57, C07C 211/37, C07C 69/74

# (54) 2,2-Difluorcyclopropyl-Derivate.

(30) Priorität: **19.07.88 DE 3824432**

(43) Veröffentlichungstag der Anmeldung:
**24.01.90 Patentblatt 90/04**

(45) Bekanntmachung des Hinweises auf die Patenterteilung:
**30.12.92 Patentblatt 92/53**

(84) Benannte Vertragsstaaten:
**BE CH DE FR GB IT LI NL**

(56) Entgegenhaltungen:
**EP-A- 0 198 192**
**EP-A- 0 318 425**
**DE-A- 2 653 189**

(73) Patentinhaber: **BAYER AG**
**W-5090 Leverkusen 1 Bayerwerk(DE)**

(72) Erfinder: **Gassen, Karl-Rudolf, Dr.**
**Auenweg 6a**
**W-5068 Odenthal(DE)**
Erfinder: **Baasner, Bernd, Dr.**
**Hambergerstrasse 27d**
**W-5090 Leverkusen 3(DE)**

EP 0 351 647 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 2,2-Difluorcyclopropyl-Derivate, mehrere Verfahren zu deren Herstellung und deren Verwendung als Zwischenprodukte zur Synthese von Verbindungen mit fungizider Wirksamkeit.

Es sind bereits bestimmte Cyclopropyl-Derivate und deren Verwendung als Zwischenprodukte zur Herstellung von Azolyl-Derivaten mit fungiziden Eigenschaften bekannt (vgl. EP- A 0 040 345 und EP-A 0 180 136). So lassen sich 1-(4-Chlorphenoxy)-2-cyclopropyl-3-(1,2,4-triazol-1-yl)-propan-2-ol, 1-(4-Chlorphenyl)-1-(1-chlor-cycloprop-1-yl)-2-(1,2,4-triazol-1-yl)-ethan-1-ol und 1-(4-Chlorphenyl)-1-[1-(2,4-dichlorphenoxy)-cycloprop-1-yl]-2-(1,2,4-triazol-1-yl)-ethan-1-ol aus entsprechenden Cyclopropyl-Derivaten herstellen und zur Bekämpfung von Pilzen verwenden. Die Wirksamkeit dieser Stoffe ist gut, läßt aber bei niedrigen Aufwandmengen in manchen Fällen zu wünschen übrig.

Außerdem sind aus der DE-A 2 653 189 Diluorcyclopropyl-Derivate bekannt, die an einem Kohlenstoffatom des Cyclopropanringes sowohl eine Carboxylgruppe als auch einen substituierten Phenylrest enthalten. Entsprechende Verbindungen, in denen statt der substituierten Phenyl-Gruppe andere Reste vorhanden sind, werden aber nicht offenbart.

Es wurden nun neue 2,2-Difluorcyclopropyl-Derivate der Formel

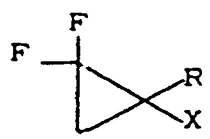

(I)

in welcher

R für Alkyl mit 1 bis 4 Kohlenstoffatomen steht oder für Benzyl steht, das im Phenylteil einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Alkoximinoalkyl mit 1 bis 4 Kohlenstoffatomen im Alkylteil und 1 bis 4 Kohlenstoffatomen im Alkoxyteil, gegebenenfalls durch Alkyl mit 1 oder 2 Kohlenstoffatomen und/oder Halogen substituiertes Phenoximinoalkyl mit 1 bis 4 Kohlenstoffatomen im Alkylteil, gegebenenfalls durch Alkyl mit 1 oder 2 Kohlenstoffatomen und/oder Halgoen substituiertes Phenyl oder gegebenenfalls durch Alkyl mit 1 oder 2 Kohlenstoffatomen und/oder Halogen substituiertes Phenoxy,

X für Hydroxymethyl, 2-Hydroxyethyl, Isocyanato, Amino oder Aminochlorid steht oder für den Rest der Formel -CO-$R^1$ steht, worin

$R^1$ für Wasserstoff, Hydroxy, Alkoxy mit 1 bis 4 Kohlenstoffatomen, Alkyl mit 1 bis 4 Kohlenstoffatomen, Chlor oder Amino steht,

gefunden.

Weiterhin wurde gefunden, daß sich 2,2-Difluorcyclopropyl-Derivate der Formel (I) herstellen lassen, indem man

a) Vinylcyclopropan-Derivate der Formel

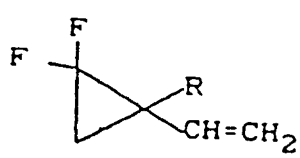

(II)

in welcher

R die oben angegebene Bedeutung hat,

entweder

α) mit einem starken Oxidationsmittel in Gegenwart eines Verdünnungsmittels umsetzt,

oder

β) mit Ozon in Gegenwart eines Verdünnungsmittels sowie mit einem Reduktionsmittel umsetzt,

oder

γ) zunächst in einer ersten Stufe mit Diboran in Gegenwart eines Verdünnungsmittels umsetzt und das dabei entstehende Produkt danach in einer zweiten Stufe mit einen starken Oxidationsmittel in Gegenwart eines Verdünnungsmittels umsetzt,

oder

b) 2,2-Difluorcyclopropyl-Derivate der Formel

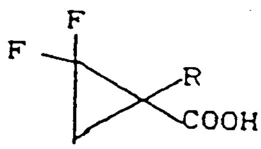

(Ia)

in welcher

R die oben angegebene Bedeutung hat

entweder

α) mit Thionylchlorid, Sulfurylchlorid oder Phosphortrichlorid gegebenenfalls in Gegenwart eines Verdünnungsmittels umsetzt,

oder

β) mit Lithium-aluminium-hydrid in Gegenwart eines Verdünnungsmittels umsetzt,

oder

γ) mit metallorganischen Verbindungen der Formel

$R^2$-Li (III)

in welcher

$R^2$ für Alkyl mit 1 bis 6 Kohlenstoffatomen steht, in Gegenwart eines Verdünnungsmittels umsetzt.

oder

c) 2,2-Difluorcyclopropyl-Derivate der Formel

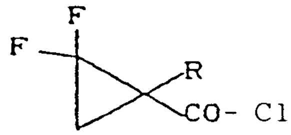

(Ib)

in welcher

R die oben angegebene Bedeutung hat,

entweder

α) mit einem Azidgruppen übertragenden Reagenz in Gegenwart eines Verdünnungsmittels umsetzt und die dabei entstehenden Verbindungen der Formel

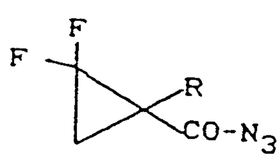

(Ic)

in welcher

R die oben angegebene Bedeutung hat,

in Gegenwart eines Verdünnungsmittels thermisch zersetzt,

oder

β) mit Alkoholen der Formel $R^3$-OH (IV)

in welcher

$R^3$ für Alkyl mit 1 bis 6 Kohlenstoffstomen steht,

gegebenenfalls in Gegenwart eines Katalysators sowie gegebenenfalls in Gegenwart eines Verdünnungsmittels umsetzt,

oder

γ) mit Ammoniak gegebenenfalls in Gegenwart eines Verdünnungsmittels umsetzt,

oder

d) 2,2-Difluorcyclopropyl-Derivate der Formel

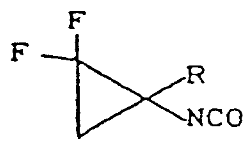

(Id)

in welcher

R die oben angegebene Bedeutung hat

mit Chlorwasserstoff-Säure in Gegenwart eines Verdünnungsmittels unsetzt und gegebenenfalls die dabei entstehenden Verbindungen der Formel

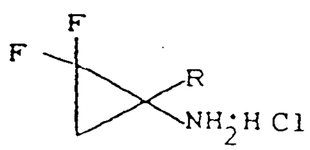

(Ie)

in welcher

R die oben angegebene Bedeutung hat und

mit Basen gegebenenfalls in Gegenwart eines Verdünnungsmittels umsetzt.

Schließlich wurde gefunden, daß sich die neuen 2,2-Difluorcyclopropyl-Derivate der Formel (I) sehr gut als Zwischenprodukte zur Herstellung von 2,2-Difluorcyclopropyl-hydroxyethyl-azolenmit fungizider Wirksamkeit verwenden lassen.

Überraschenderweise zeigen die aus den erfindungsgemäßen 2,2-Difluorcyclopropyl-Derivaten der Formel (I) herstellbaren 2,2-Difluorcyclopropyl-hydroxyethyl-azole eine bessere fungizide Wirksamkeit als 1-(4-Chlorphenoxy)-2-cyclopropyl-3-(1,2,4-triazol-1-yl)-propan-2-ol, 1-(4-Chlorphenyl)-1-(1-chlor-cycloprop-1-yl)-2-(1,2,4-triazol-1-yl)-ethan-1-ol und 1-(4-Chlorphenyl)-1-[1-(2,4-dichlorphenoxy)-cycloprop-1-yl]-2-(1,2,4-triazol-1-yl)-ethan-1-ol, welches konstitutionell ähnliche, vorbekannte Wirkstoffe gleicher Wirkungsrichtung sind.

Die erfindungsgemäßen 2,2-Difluorcyclopropyl-Derivate sind durch die Formel (I) allgemein definiert. Bevorzugt sind diejenigen Verbindungen, in denen

R für Methyl, Ethyl, Isopropyl, tert.-Butyl steht, oder für Benzyl steht, das im Phenylteil einfach oder zweifach, gleichartig oder verschieden substituiert sein kann durch Fluor, Chlor und/oder Methyl,

und

X für Hydroxymethyl, 2-Hydroxymethyl, Isocyanato, Amino, Amino-hydrochlorid steht oder für den Rest der Formel -CO-$R^1$ steht, worin

$R^1$ für Wasserstoff, Hydroxy, Alkoxy mit 1 bis 4 Kohlenstoffatomen, Alkyl mit 1 bis 4 Kohlenstoffatomen, Chlor oder Amino steht.

Besonders bevorzugt sind diejenigen 2,2-Difluorcyclopropyl-Derivate der Formel (I), in denen

R für Methyl, Ethyl, gegebenenfalls einfach oder zweifach durch Fluor, Chlor und/oder Methyl substituiertes Benzyl steht,

und

X für Hydroxymethyl, 2-Hydroxyethyl, Isocyanato, Amino, Amino-hydrochlorid steht oder für den Rest der Formel
-CO-R[1] steht, worin

R[1] für Wasserstoff, Hydroxy, Methoxy, Ethoxy, Isopropoxy, n-Butoxy, Methyl, Ethyl, Isopropyl, n-Butyl, Chlor oder Amino steht.

Verwendet man 2,2-Difluor-1-methyl-1-vinyl-cyclopropan als Ausgangsstoff und Kaliumpermanganat als Oxidationsmittel, so kann der Verlauf des erfindungsgemäßen Verfahrens (a, Variante α) durch das folgende Formelschema veranschaulicht werden:

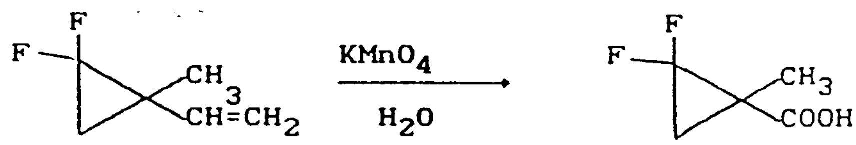


Verwendet man 2,2-Difluor-1-methyl-1-vinyl-cyclopropan als Ausgangsstoff und Ozon als Reaktionskomponente, so kann der Verlauf des erfindungsgemäßen Verfahrens (a, Variante β) durch das folgende Formeschema veranschaulicht werden:

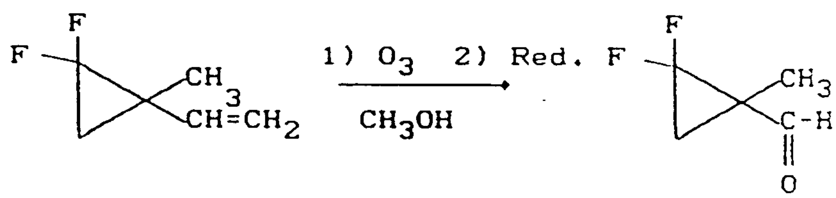


Verwendet man 2,2-Difluor-1-methyl-1-vinyl-cyclopropan als Ausgangsstoff und Diboran sowie anschließend Wasserstoffperoxid als Reaktionskomponenten, so kann der Verlauf des erfindungsgemäßen Verfahrens (a, Variante γ) durch das folgende Formelschema veranschaulicht werden:

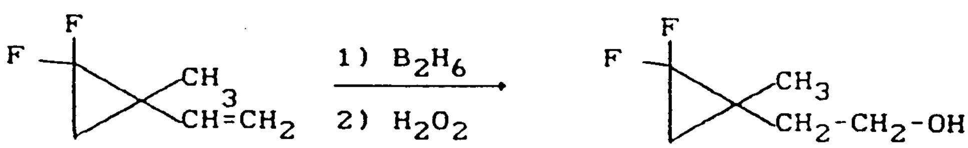


Verwendet man 2,2-Difluor-1-methyl-1-cyclopropancarbonsäure als Ausgangsstoff und Thionylchlorid als Halogenierungsmittel, so kann der Verlauf des erfindungsgemäßen Verfahrens (b, Variante α) durch das folgende Formeschema veranschaulicht werden:

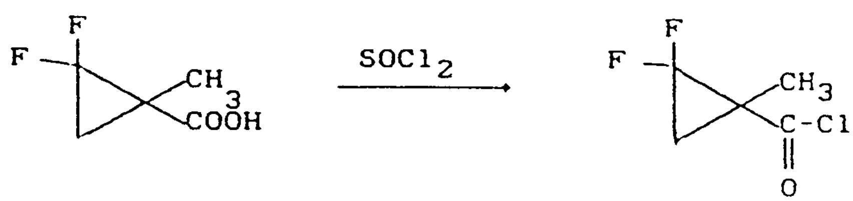


Verwendet man 2,2-Difluor-1-methyl-1-cyclopropancarbonsäure als Ausgangsstoff und Lithiumaluminiumhydrid als komplexes Hydrid, so kann der Verlauf des erfindungsgemäßen Verfahrens (b, Variante β) durch das folgende Formelschema veranschaulicht werden:

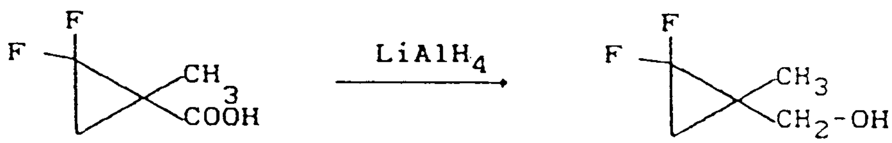


Verwendet man 2,2-Difluor-1-methyl-cyclopropancarbonsäure als Ausgangsstoff und Methyl-lithium als metallorganische Verbindung, so kann der Verlauf des erfindungsgemäßen Verfahrens (b, Variante γ) durch das folgende Formeschema veranschaulicht werden:

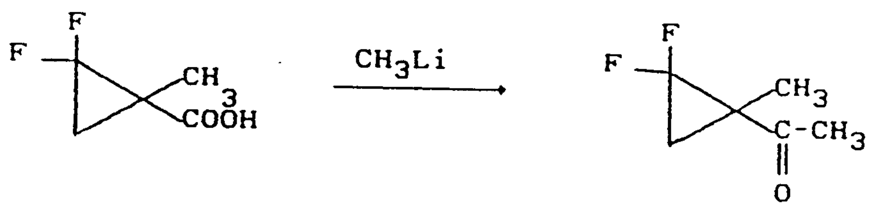


Verwendet man 2,2-Difluor-1-methyl-1-cyclopropancarbonsäure als Ausgangsstoff und Trimethylsilylazid als Reaktionskomponente, so kann der Verlauf des erfindungsgemäßen Verfahrens (c, Variante α) durch das folgende Formelschema veranschaulicht werden:

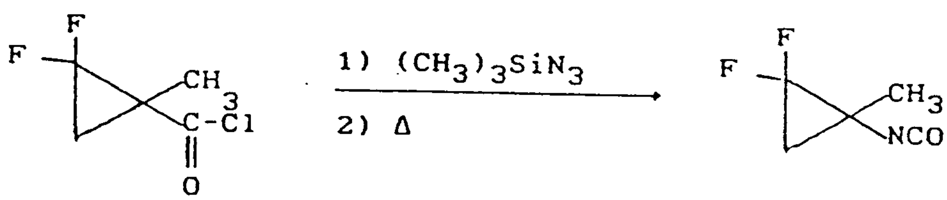


Verwendet man 2,2-Difluor-1-methyl-cyclopropan-carbonsäurechlorid als Ausgangsstoff und Ethanol als Reaktionskomponente, so kann der Verlauf des erfindungsgemäßen Verfahrens (c, Variante β) durch das folgende Formelschema veranschaulicht werden:

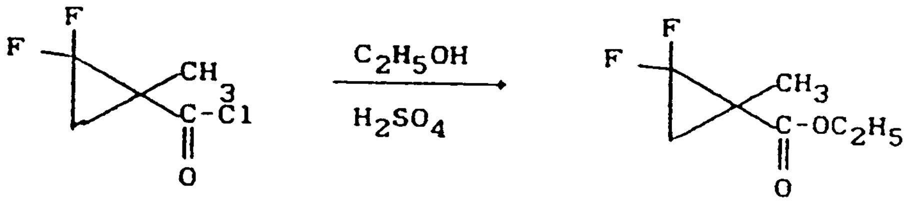


Verwendet man 2,2-Difluor-1-methyl-1-cyclopropan-carbonsäure-chlorid als Ausgangsstoff und Ammoniak als Reaktionskomponente, so kann der Verlauf des erfindungsgemäßen Verfahrens (c, Variante γ) durch das folgende Formelschema veranschaulicht werden:

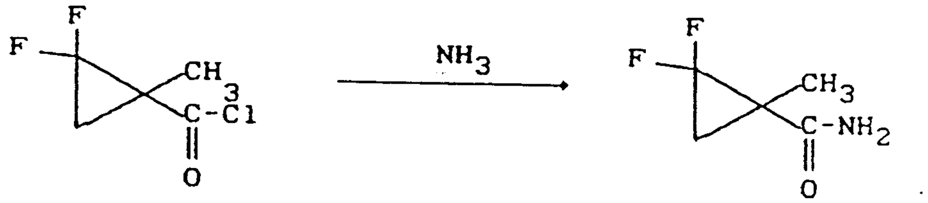


Verwendet man 2,2-Difluor-1-methyl-cyclopropyl-isocyanat als Ausgangsstoff und konzentrierte Salzsäure als Reaktionskomponente, so kann der Verlauf des erfindungsgemäßen Verfahrens (d) durch das folgende Formelschema veranschaulicht werden:

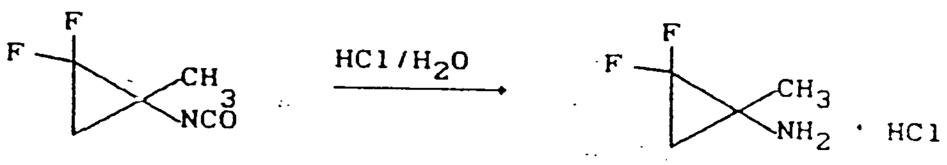


Die bei der Durchführung des erfindungsgemäßen Verfahrens (a) als Ausgangsstoffe benötigten Vinylcyclopropan-Derivate sind durch die Formel (II) allgemein definiert. In dieser Formel steht R vorzugsweise für diejenigen Reste, die bereits im Zusammenhang mit der Beschreibung der erfindungsgemäßen Stoffe der Formel (I) vorzugsweise für R genannt wurden.

Die Vinylcyclopropan-Derivate der Formel (II) sind bekannt oder lassen sich nach prinzipiell bekannten Verfahren herstellen (vgl. Liebigs Ann. Chem. 710, 17-35 (1967) und Chem. Ber. 109, 2351-2369 (1976)).

Als starke Oxidationsmittel kommen bei der Durchführung des erfindungsgemäßen Verfahrens (a, Variante $\alpha$) alle diejenigen Oxidationsmittel in Betracht, die zur Spaltung von olefinischen Doppelbindungen geeignet sind. Vorzugsweise verwendbar ist Kaliumpermanganat.

Als Verdünnungsmittel kommen bei der Durchführung der Variante $\alpha$ des erfindungsgemäßen Verfahrens (a) alle für derartige Umsetzungen üblichen Lösungsmittel in Frage. Vorzugsweise verwendbar ist Wasser.

Die Temperaturen können bei der Durchführung des erfindungsgemäßen Vefahrens (a, Variante $\alpha$) innerhalb eines bestimmten Bereiches variiert werden. Im allgemeinen arbeitet man bei Temperaturen zwiscne 0 ° C und 60 ° C, vorzugsweise zwischen 10 ° C und 50 ° C.

Bei der Durchführung des erfindungsgemäßen Verfahrens (a, Variante $\alpha$) arbeitet man ebenso wie bei den anderen in dieser Anmeldung beschriebenen Verfahren im allgemeinen unter Normaldruck. Es ist jedoch jeweils auch möglich, unter erhöhtem oder vermindertem Druck zu arbeiten.

Bei der Durchführung des erfindungsgemäßen Verfahrens (a, Variante $\alpha$) setzt man auf 1 Mol an Vinylcyclopropan-Derivat der Formel (II) im allgemeinen 2 bis 3 Mol an starkem Oxidationsmittel ein. Die Aufarbeitung erfolgt nach üblichen Methoden.

Als Reduktionsmittel kommen bei der Durchführung des erfindungsgemäßen Verfahrens (a, Variante $\beta$) alle für derartige Ozonolysen üblichen Reduktionsmittel in Frage. Vorzugsweise verwendbar sind Triphenylphosphin, Trimethylphosphit, Dimethylsulfid und Wasserstoff in Gegenwart eines Katalysators.

Als Verdünnungsmittel kommen bei der Durchführung des erfindungsgemäßen Verfahrens (a, Variante $\beta$) alle für derartige Ozonolysen üblichen Solventien in Betracht. Vorzugsweise verwendbar sind Alkohole, wie Methanol oder Ethanol.

Die Reaktionstemperaturen lassen sich bei der Durchführung des erfindungsgemäßen Verfahrens (a, Variante $\beta$) innerhalb eines bestimmten Bereiches variieren. Im allgemeinen arbeitet man bei Temperaturen zwischen - 80 ° C und -20 ° C, vorzugsweise zwischen -80 ° C und - 40 ° C.

Bei der Durchführung des erfindungsgemäßen Verfahrens (a, Variante $\beta$) leitet man Ozon durch das Reaktionsgemisch bis zur Beendigung der Umsetzung. Die Aufarbeitung erfolgt nach üblichen Methoden. Im allgemeinen geht man so vor, daß man einen Überschuß an Reduktionsmittel hinzugibt und zunächst weiter unter Kühlung rührt. Nach dem Erwärmen auf Raumtemperatur verdünnt man mit Wasser, extrahiert mit einem in Wasser wenig löslichen organischen Solvens, trocknet die vereinigten organischen Phasen und destilliert.

Das bei der Durchführung des erfindungsgemäßen Verfahrens (a, Variante γ) benötigte Diboran wird im allgemeinen frisch erzeugt, z.B. durch Umsetzung von Bortrifluorid-etherat mit Natriumborhydrid in Gegenwart eines inerten organischen Verdünnungsmittels, wie z.B. Diglyme. Das so erzeugte Diboran wird direkt weiter umgesetzt.

Als Verdünnungsmittel kommen bei der Durchführung der ersten Stufe des erfindungsgemäßen Verfahrens (a, Variante γ) alle für derartige Umsetzungen üblichen inerten organischen Solventien in Frage. Vorzugsweise verwendbar sind Ether, wie Diethylether, Dioxan oder Tetrahydrofuran.

Bei der Durchführung der ersten Stufe des erfindungsgemäßen Verfahrens (a, Variante γ) arbeitet man gegebenenfalls unter Schutzgasatmosphäre, vorzugsweise unter Stickstoff oder unter Argon.

Die Reaktionstemperaturen können bei der Durchführung des erfindungsgemäßen Verfahrens (a, Variante γ) sowohl in der ersten als auch in der zweiten Stufe innerhalb eines bestimmten Breiches variiert werden. Im allgemeinen arbeitet man bei Temperaturen zwischen 0 ° C und 60 ° C, vorzugsweise zwischen 10 ° C und 50 ° C.

Bei der Durchführung der ersten Stufe des erfindungsgemäßen Verfahrens (a, Variante γ) setzt man auf 1 Mol an Vinylcyclopropan-Derivat der Formel (II) im allgemeinen eine stöchiometrische Menge oder auch einen Unterschuß an Diboran ein. Nach beendeter Umsetzung geht man im allgemeinen so vor, daß man überschüssiges Vinylcyclopropan-Derivat der Formel (II) und Lösungsmittel unter vermindertem Druck entfernt Der Rückstand wird direkt weiter umgesetzt.

Als Verdünnungsmittel kommen auch bei der Durchführung der zweiten Stufe des erfindungsgemäßen Verfahrens (a, Variante γ) vorzugsweise Ether, wie Diethylether, Tetrahydrofuran und Dioxan in Frage.

Als starke Oxidationsmittel kommen bei der Durchführung der zweiten Stufe des erfindungsgemäßen Verfahrens (a, Variante γ) alle üblichen starken Oxidationsmittel in Frage. Vorzugsweise verwendbar ist Wasserstoffperoxid.

Das Wasserstoffperoxid kann bei der Durchführung des erfindungsgemäßen Verfahrens (a, Variante γ) in Form von verdünnten wäßrigen Lösungen und in Gegenwart von wäßriger Alkalilauge, wie z.B. wäßriger Natronlauge, eingesetzt werden.

Bei der Durchführung der zweiten Stufe des erfindungsgemäßen Verfahrens (a, Variante γ) geht man im allgemeinen so vor, daß man das aus der ersten Stufe erhaltene Produkt in einem Lösungsmittel aufnimmt und nacheinander jeweils mit einem Überschuß an wäßriger Alkalilauge und wäßriger Wasserstoffperoxid-Lösung oder einem ähnlichen Oxidationsmittel versetzt. Zur Aufarbeitung geht man im allgemeinen so vor, daß man mehrfach mit einem in Wasser wenig löslichen organischen Lösungsmittel extrahiert, die vereinigten organischen Phasen trocknet und unter vermindertem Druck destilliert.

Die bei der Durchführung des erfindungsgemäßen Verfahrens (b) als Ausgangsstoffe benötigten 2,2-Difluorcyclopropyl-Derivate sind durch die Formel (Ia) allgemein definiert. In dieser Formel hat R vorzugsweise diejenigen Bedeutungen, die bereits im Zusammenhang mit der Beschreibung der erfindungsgemäßen Stoffe der Formel (I) vorzugsweise für diesen Rest genannt wurden.

Die 2,2-Difluorcyclopropyl-Derivate der Formel (Ia) lassen sich nach dem erfindungsgemäßen Verfahren (a, Variante α) herstellen.

Als Verdünnungsmittel kommen bei der Durchführung des erfindungsgemäßen Verfahrens (b, Variante α) alle für derartige Reaktionen üblichen inerten organischen Solventien in Betracht. Vorzugsweise wird das jeweilige Halogenierungsmittel gleichzeitig auch als Verdünnungsmittel benutzt.

Die Reaktionstemperaturen können bei der Durchführung des erfindungsgemäßen Verfahrens (b, Variante α) innerhalb eines größeren Bereiches variiert werden. Im allgemeinen arbeitet man bei Temperaturen zwischen 0 ° C und 150 ° C, vorzugsweise zwischen 20 ° C und 80 ° C.

Bei der Durchführung des erfindungsgemäßen Verfahrens (b, Variante α) setzt man auf 1 Mol 2,2-Difluorcyclopropyl-Derivat der Formel (Ia) 1 bis 2 Äquivalente oder auch einen größeren Überschuß an Chlorierungsmittel ein. Die Aufarbeitung erfolgt nach üblichen Methoden.

Als Verdünnungsmittel kommen bei der Durchführung des erfindungsgemäßen Verfahrens (b, Variante β) Vorzugsweise Ether, wie Diethylether, Dioxan oder Tetrahydrofuran, in Frage.

Die Reaktionstemperaturen können bei der Durchführung des erfindungsgemäßen Verfahrens (b, Variante β) innerhalb eines größeren Bereiches variiert werden. Im allgemeinen arbeitet man bei Temperaturen zwischen -10 ° C und +100 ° C, vorzugsweise zwischen 10 ° C und 70 ° C.

Bei der Durchführung des erfindungsgemäßen Verfahrens (b, Variante β) setzt man auf 1 Mol an 2,2-Difluorcyclopropyl-Derivatder Formel (Ia) im allgemeinen einen Überschuß an Lithium-aluminium-Hydrid ein. Die Aufarbeitung erfolgt nach üblichen Methoden. Im allgemeinen geht man in der Weise vor, daß man durch Zugabe von Wasser und verdünnter anorganischer Säure schonend hydrolysiert, dann mit einem Solvens extrahiert, die vereinigten organischen Phasen trocknet und destilliert.

Die bei der Durchführung des erfindungsgemäßen Verfahrens (b, Variante γ) als Reaktionskomponenten

benötigten metallorganischen Verbindungen der Formel (III) sind allgemein bekannte Verbindungen der organischen Chemie.

Als Verdünnungsmittel kommen bei der Durchführung des erfindungsgemäßen Verfahrens (b, Variante $\gamma$) alle für derartige Umsetzungen üblichen inerten organischen Solventien in Frage. Vorzugsweise verwendbar sind Ether, wie Diethylether, Dioxan oder Tetrahydrofuran.

Die Reaktionstemperaturen können bei der Durchführung des erfindungsgemäßen Verfahrens (b, Variante $\gamma$) innerhalb eines größeren Bereiches variiert werden. Im allgemeinen arbeitet man bei Temperaturen zwischen -100 ° C und +50 ° C, vorzugsweise zwischen -78 ° C und 0 ° C.

Bei der Durchführung des erfindungsgemäßen Verfahrens (b, Variante $\gamma$) arbeitet man unter Schutzgasatmosphäre, wie z.B. unter Argon oder Stickstoff.

Bei der Durchführung des erfindungsgemäßen Verfahrens (b, Variante $\gamma$) setzt man auf 1 Mol an 2,2-Difluorcyclopropyl-Derivatder Formel (Ia) im allgemeinen 1,5 bis 3,0 Mol, vorzugsweise 2,0 Mol an metallorganischer Verbindung der Formel (III) ein. Die Aufarbeitung erfolgt nach üblichen Methoden. Im allgemeinen geht man so vor, daß man das Reaktionsgemisch auf Eis und anorganische Säure gibt, die organische Phase abtrennt, die wäßrige Phase mit einem mit Wasser wenig mischbaren organischen Lösungsmittel extrahiert, die vereinigten organischen Phasen trocknet und destilliert.

Die bei der Durchführung des erfindungsgemäßen Verfahrens (c, Variante $\alpha$) als Ausgangsstoffe benötigten 2,2-Difluorcyclopropyl-Derivate sind durch die Formel (Ib) allgemein definiert. In dieser Formel hat R vorzugsweise diejenigen Bedeutungen, die bereits im Zusammenhang mit der Beschreibung der erfindungsgemäßen Stoffe der Formel (I) vorzugsweise für den Rest R genannt wurden.

Die 2,2-Difluorcyclopropyl-Derivate der Formel (Ib) lassen sich nach dem erfindungsgemäßen Verfahren (b, Variante $\alpha$) herstellen.

Als Reagenzien zur Übertragung von Azid-Gruppen kommen bei der Durchführung des erfindungsgemäßen Verfahrens (c, Variante $\alpha$) alle für derartige Umsetzungen üblichen Stoffe in Betracht. Vorzugsweise verwendbar ist Trimethylsilylazid.

Als Verdünnungsmittel kommen bei der Durchführung des erfindungsgemäßen Verfahrens (c, Variante $\alpha$) sowohl bei der Herstellung der Verbindungen der Formel (Ic) als auch bei deren thermischer Zersetzung alle für derartige Umsetzungen übliche inerten organischen Solventien in Frage. Vorzugsweise verwendbar sind aliphatische oder aromatische Kohlenwasserstoffe, wie Hexan, Benzol, Xylol oder Toluol.

Die Reaktionstemperaturen können bei der Durchführung des erfindungsgemäßen Verfahrens (c, Variante $\alpha$) innerhalb eines größeren Bereiches variiert werden. Im allgemeinen arbeitet man bei der Herstellung der Verbindungen der Formel (Ic) bei Temperaturen zwischen 0 ° C und 30 ° C, vorzugsweise zwischen 5 ° C und 25 ° C. Bei der anschließenden thermischen Zersetzung der Verbindungen der Formel (Ic) arbeitet man im allgemeinen zwischen 20 ° C und 120 ° C, vorzugsweise zwischen 25 ° C und 100 ° C.

Bei der Durchführung des erfindungsgemäßen Verfahrens (c, Variante $\alpha$) setzt man auf 1 Mol an 2,2-Difluorcyclopropyl-Derivatder Formel (Ib) vorzugsweise 1 bis 1,5 Mol an einem Azidgruppen übertragenden Reagenz ein. Das dabei intermediär entstehende Produkt der Formel (Ic) wird ohne Isolierung durch langsame Steigerung der Temperatur des Reaktionsgemisches thermisch zersetzt Die anschließende Aufarbeitung erfolgt nach üblichen Methoden. Im allgemeinen geht man so vor, daß man das Reaktionsgemisch einer fraktionierten Destillation unterwirft.

Die bei der Durchführung des erfindungsgemäßen Verfahrens (c, Variante $\beta$) als Reaktionskomponenten benötigten Alkohole der Formel (IV) sind allgemein bekannte Verbindungen der organischen Chemie.

Als Katalysatoren kommen bei der Durchführung des erfindungsgemäßen Verfahrens (c, Variante $\beta$) alle für die Herstellung von Estern aus Säuren oder Säurechloriden üblichen Reaktionsbeschleuniger in Frage. Vorzugsweise verwendbar sind anorganische Säuren, wie Schwefelsäure, oder auch starke organische Säuren, wie p-Toluolsulfonsäure. Ebenfalls verwendbar sind anorganische Basen, wie Natriumhydroxid, oder auch organische Basen, wie Pyridin oder tertiäre Amine.

Als Verdünnungsmittel kommen bei der Durchführung des erfindungsgemäßen Verfahrens (c, Variante $\beta$) alle für derartige Umsetzungen üblichen organischen Solventien in Betracht. Vorzugsweise fungiert im Überschuß eingesetzter Alkohol der Formel (IV) auch als Lösungsmittel.

Die Reaktionstemperaturen können bei der Durchführung des erfindungsgemäßen Verfahrens (c, Variante $\beta$) innerhalb eines größeren Bereiches variiert werden. Im allgemeinen arbeitet man bei Temperaturen zwischen 0 ° C und 150 ° C, vorzugsweise zwischen 20 ° C und 140 ° C.

Bei der Durchführung des erfindungsgemäßen Verfahrens (c, Variante $\beta$) setzt man auf 1 Mol an 2,2-Difluorcyclopropyl-Derivatder Formel (Ib) vorzugsweise 1 bis 3 Mol oder auch einen größeren Überschuß an Alkohol der Formel (IV) sowie eine katalytische Menge an Reaktionsbeschleuniger ein. Die Aufarbeitung erfolgt nach üblichen Methoden.

Als Verdünnungsmittel kommen bei der Durchführung des erfindungsgemäßen Verfahrens (c, Variante

γ) alle für derartige Umsetzungen üblichen inerten organischen Solventien in Frage. Vorzugsweise verwendbar sind Ether, wie Diethylether, Dioxan und Tetrahydrofuran.

Die Reaktionstemperaturen können auch bei der Durchführung des erfindungsgemäßen Verfahrens (c, Variante γ) innerhalb eines bestimmten Bereiches variiert werden. Im allgemeinen arbeitet man bei Temperaturen zwischen 0 ° C und 60 ° C, vorzugsweise zwischen 10 ° C und 40 ° C.

Bei der Durchführung des erfindungsgemäßen Verfahrens (c, Variante γ) setzt man 2,2-Difluorcyclopropyl-Derivate der Formel (Ib) mit einem Überschuß an Ammoniak um. Zweckmäßigerweise geht man dabei so vor, daß man das 2,2-Difluorcyclopropyl-Derivat der Formel (Ib) in Losung vorlegt und bis zur Sättigung Ammoniak-Gas einleitet Die Aufarbeitung erfolgt nach üblichen Methoden. Im allgemeinen geht man so vor, daß man das Reaktionsgemisch unter vermindertem Druck einengt und das dabei verbleibende Produkt mit Wasser verrührt, absaugt und trocknet.

Die bei der Durchführung des erfindungsgemäßen Verfahrens (d) als Ausgangsstoffe benötigten 2,2-Difluorcyclopropyl-Derivate sind durch die Formel (Id) allgemein definiert. In dieser Formel hat R vorzugsweise diejenigen Bedeutungen, die bereits im Zusammenhang mit der Beschreibung der erfindungsgemäßen Stoffe der Formel (I) vorzugsweise für den Rest R genannt wurden.

Die 2,2-Difluorcyclopropyl-Derivate der Formel (Id) lasen sich nach dem erfindungsgemäßen Verfahren (c, Variante α) herstellen.

Als Verdünnungsmittel kommen bei der Durchführung des erfindungsgemäßen Verfahrens (d) alle für derartige Umsetzungen üblichen organischen Solventien in Frage. Vorzugsweise verwendbar sind aliphatische oder aromatische Kohlenwassertoffe, wie Hexan, Benzol, Toluol oder Xylol.

Die Reaktionstemperaturen können bei der Durchführung des erfindungsgemäßen Verfahrens (d) innerhalb eines bestimmten Breiches variiert werden. Im allgemeinen arbeitet man bei Temperaturen zwischen 0 ° C und 150 ° C, vorzugsweise zwischen 10 ° C und 100 ° C.

Bei der Durchführung des erfindungsgemäßen Verfahrens (d) setzt man auf 1 Mol an 2,2-Difluorcyclopropyl-Derivat der Formel (Id) vorzugsweise 1 bis 5 Mol an Chlorwasserstoffsäure in Form einer wäßrigen Lösung ein. Die Aufarbeitung erfolgt nach üblichen Methoden. Im allgemeinen geht man in der Weise vor, daß man das Reaktionsgemisch unter vermindertem Druck einengt und den verbleibenden Rückstand gegebenenfalls nach üblichen Methoden reinigt.

Sofern bei der Durchführung des erfindungsgemäßen Verfahrens (d) die Herstellung der freien Amine beabsichtigt ist, versetzt man die Verbindungen der Formel (Ie) mit Basen gegebenenfalls in Gegenwart eines Verdünnungsmittels. Als Basen kommen hierbei Vorzugsweise wäßrige Alkalilaugen, wie Natronlauge oder Kalilauge in Frage. Als Verdünnungsmittel kommen inerte organischen Solventien oder Wasser in Betracht.

Die Reaktionstemperaturen können bei der Herstellung der freien Amine nach dem erfindungsgemäßen Verfahren (d) innerhalb eines größeren Bereiches variiert werden. Im allgemeinen arbeitet man bei Temperaturen zwischen -10 ° C und +50 ° C, vorzugsweise zwischen 0 ° C und +30 ° C.

Bei der Herstellung der freien Amine nach dem erfindungsgemäßen Verfahren (d) setzt man die Verbindungen der Formel (Ie) mit einem Überschuß an Base um. Die Aufarbeitung erfolgt nach üblichen Methoden. Im allgemeinen geht man in der Weise vor, daß man das Reaktionsgemisch gegebenenfalls nach vorherigem Umsetzen mit Wasser mit einem mit Wasser wenig mischbaren organischen Solvens extrahiert, die vereinigten organischen Phasen trocknet und einengt und den verbleibenden Rückstand destilliert.

Die erfindungsgemäßen 2,2-Difluorcyclopropyl-Derivate der Formel (I) eignen sich als Zwischenprodukte zur Synthese von Pflanzenschutzmitteln, insbesondere zur Herstellung von Stoffen mit fungizider Wirksamkeit.

So lassen sich z.B. 2,2-Difluorcyclopropyl-hydroxyethyl-triazole der Formel

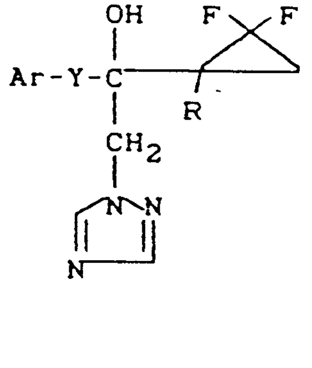

(V)

in welcher

R die oben angegebene Bedeutung hat,

Ar für gegebenenfalls substituiertes Aryl steht und

Y für die Gruppierungen $-OCH_2-$, $-SCH_2-$, $-CH_2-CH_2-$ oder $-CH=CH-$ steht,

herstellen, indem man

e) Methyl-cyclopropyl-ketone der Formel

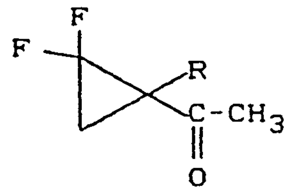

(If)

in welcher

R die oben angegebene Bedeutung hat,

mit Chlorierungsmitteln oder Bromierungsmitteln, wie Sulfurylchlorid, Sulfurylbromid oder Brom, in Gegenwart eines Verdünnungsmittels, wie Methylenchlorid, Chloroform oder Tetrachlorkohlenstoff, bei Temperaturen zwischen -10°C und +60°C, vorzugsweise zwischen 0°C und 40°C, umsetzt und die dabei entstehenden Halogenketone der Formel

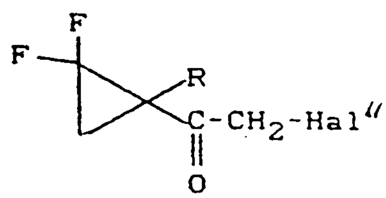

(VI)

in welcher

R die oben angegebene Bedeutung hat

und

Hal" für Chlor oder Brom steht,

mit Verbindungen der Formel

Ar-Z-H (VII)

in welcher

Ar die oben angegebene Bedeutung hat und

Z für Sauerstoff oder Schwefel steht,

in Gegenwart eines Säurebindemittels und gegebenenfalls in Gegenwart eines Verdünnungsmittels bei Temperaturen zwischen 0°C und 150°C, vorzugsweise zwischen 20°C und 130°C, umsetzt und die dabei entstehenden Cyclopropyl-ketone der Formel

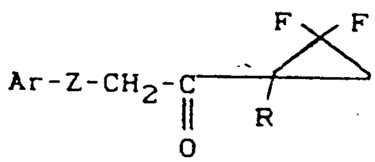

(VIII)

in welcher

Ar, R und Z die oben angegebene Bedeutung haben, entweder

α) mit Dimethyloxosulfonium-methylid der Formel

$$\overset{\delta^{\ominus}\,\delta^{\ominus}}{(CH_3)_2SOCH_2} \qquad (IX)$$

oder

β) mit Dimethylsulfonium-methylid der Formel

$$\overset{\delta^{\ominus}\,\delta^{\ominus}}{(CH_3)_2S\ CH_2} \qquad (X)$$

in Gegenwart eines Verdünnungsmittels bei Temperaturen zwischen 0 ° C und 100 ° C, vorzugsweise zwischen 10 ° C und 60 ° C, umsetzt und schließlich die dabei entstehenden Oxirane der Formel

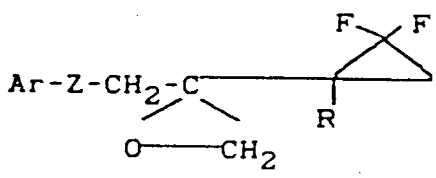

(XI)

in welcher

Ar, R und Z die oben angegebene Bedeutung haben, mit 1,2,4-Triazol der Formel

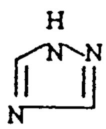

(XII)

in Gegenwart eines Säurebindemittels und in Gegenwart eines Verdünnungsmittels bei Temperaturen zwischen 0 ° C und 200 ° C, vorzugsweise zwischen 50 ° C und 150 ° C, umsetzt,

oder indem man

f) Methyl-cyclopropyl-ketone der Formel

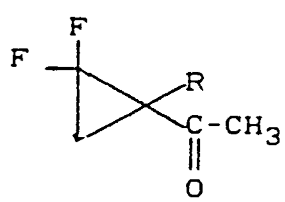

(If)

in welcher

R die oben angegebene Bedeutung hat,

mit Aldehyden der Formel

Ar-CHO (XIII)

in welcher

Ar die oben angegebene Bedeutung hat,

in Gegenwart eines Katalystors, wie Natriumhydroxid oder Kaliumhydroxid, sowie in Gegenwart eines

Verdünnungsmittels, wie Methanol, Ethanol, Isopropanol, n-Butanol oder tert.-Butanol, bei Temperaturen zwischen 0 °C und 100 °C, vorzugsweise zwischen 10 °C und 80 °C, umsetzt und gegebenenfalls die dabei entstehenden Cyclopropyl-ketone der Formel

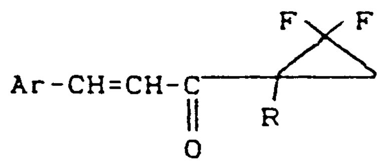
(XIV)

in welcher
R und Ar die oben angegebene Bedeutung haben,
in Gegenwart eines Hydrierkatalysators und in Gegenwart eines Verdünnungsmittels mit Wasserstoff hydriert und schließlich die so erhaltenen Cyclopropyl-ketone der Formel

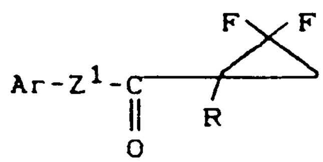
(XV)

in welcher
Ar und R die oben angegebenen Bedeutungen haben und
$Z^1$ für die Gruppierungen -CH = CH- oder $-CH_2-CH_2-$steht,
α) mit Dimethyloxosulfonium-methylid der Formel

$$\overset{\delta^{\oplus}\delta^{\ominus}}{(CH_3)_2SOCH_2} \qquad (IX)$$

oder
β) mit Dimethylsulfonium-methylid der Formel

$$\overset{\delta^{\oplus}\delta^{\ominus}}{(CH_3)_2S\ CH_2} \qquad (X)$$

in Gegenwart eines Verdünnungsmittels bei Temperaturen zwischen 0 °C und 100 °C, vorzugsweise zwischen 10 °C und 60 °C, umsetzt und schließlich die dabei entstehenden Oxirane der Formel

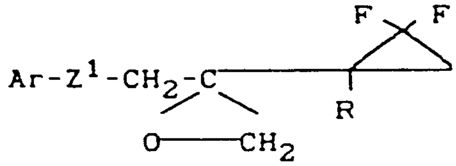
(XVI)

in welcher
Ar, R und $Z^1$ die oben angegebene Bedeutung haben,

mit 1,2,4-Triazol der Formel

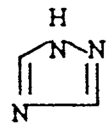

(XII)

in Gegenwart eines Säurebindemittels und in Gegenwart eines Verdünnungsmittels bei Temperaturen zwischen 0 °C und 200 °C, vorzugsweise zwischen 50 °C und 150 °C, umsetzt.

Weiterhin lassen sich Hydroxyalkinyl-azolyl-Derivate der Formel

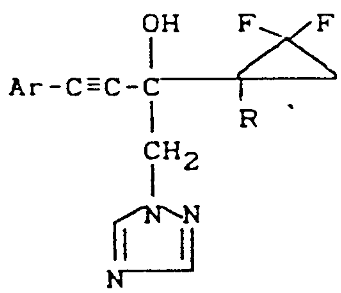

(XVII)

in welcher

Ar und R die oben angegebene Bedeutung haben,

herstellen, indem man

g) 2,2-Difluorcyclopropyl-Derivate der Formel

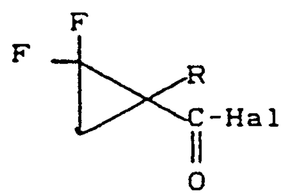

(Ib)

in welcher

R und Hal die oben angegebene Bedeutung haben,

mit Acetyl-Derivaten der Formel

Ar-C≡CH (XVIII)

in welcher

Ar die oben angegebene Bedeutung hat,

in Gegenwart eines Katalysators, wie Kupfer-(I)-bromid, und in Gegenwart eines Säurebindemittels, wie Natriumhydroxid oder Kaliumhydroxid, und in Gegenwart eines Verdünnungsmittels, wie Toluol, bei Temperaturen zwischen 0 °C und 150 °C, vorzugsweise zwischen 20 °C und 120 °C, umsetzt und die dabei entstehenden Cyclopropylketone der Formel

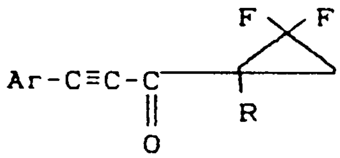

(XIX)

in welcher

Ar und R die oben angegebene Bedeutung haben,

mit Dimethylsulfonium-methylid der Formel

$$\overset{\delta^{\oplus}}{(CH_3)_2S}\;\overset{\delta^{\ominus}}{CH_2} \qquad (X)$$

in Gegenwart eines Verdünnungsmittels bei Temperaturen zwischen 0 ° C und 100 ° C, vorzugsweise zwischen 10 ° C und 60 ° C, umsetzt und schließlich die dabei entstehenden Oxirane der Formel

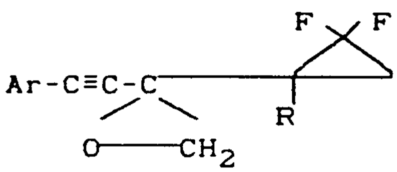

(XX)

in welcher

Ar und R die oben angegebene Bedeutung haben,

mit 1,2,4-Triazol der Formel

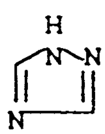

(XII)

in Gegenwart eines Säurebindemittels und in Gegenwart eines Verdünnungsmittels bei Temperaturen zwischen 0 ° C und 200 ° C, vorzugsweise zwischen 50 ° C und 150 ° C, umsetzt.

Die übrigen 2,2-Difluorcyclopropyl-Derivate der Formel (I) lassen sich in entsprechender Weise als Zwischenprodukte zur Synthese von Pflanzenschutzmitteln, insbesondere von Stoffen mit fungizider Wirksamkeit verwenden.

Die 2,2-Difluorcyclopropyl-hydroxyethyl-triazole der Formel (V) und die Hydroxalkinyl-azolyl-Derivate der Formel (XVII) weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;

Pseudomonas-Arten, wie Pseudomonas lachrymans;

Erwinia-Arten, wie Erwinia amylovora;

Pythium-Arten, wie Pythium ultimum;

Phytophthora-Arten, wie Phytophthora infestans, Pseudoperonospora-Arten, wie Pseudoperonospora humuli oder Pseudoperonospora cubense;

Plasmopara-Arten, wie Plasmopara viticola;

Peronospora-Arten, wie Peronospora pisi oder P. brassicae;

Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus, Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe der Formeln (V) und (XVII) in den zur Bekämpfung von Pflanzenkrankheiten notwendigen Konzentrationen erlaubt eine Behandlung von oberirdischen Pflanzenteilen, von Pflanz- und Saatgut und des Bodens.

Die Wirkstoffe der Formeln (V) und (XVII) eignen sich insbesondere zur Bekämpfung von Getreidekrankheiten, wie Erysiphe graminis, Puccinia recondita, Cochliobolus sativus, Pyrenophora teres, Leptospaeria nodorum und Gerstenmehltau; ferner von Reiskranheiten, wie Pyricularia oryzae und Pellicularia sasakii; sowie von Venturia-Arten und Gurkenmehltau. Die Stoffe besitzen außerdem eine sehr gute in-vitro-Wirkung.

Die Wirkstoffe der Formeln (V) und (XVII) können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Träger stoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsaure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe der Formeln (V) und (XVII) können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe der Formeln (V) und (XVII) können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren.

Es kann auch das Saatgut der Pflanzen behandelt werden.

Die Aufwandmenge an Wirkstoffen der Formeln (V) und (XVII) kann je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung der erfindungsgemäßen 2,2-Difluorcyclopropyl-Derivate der Formel (I) und deren Verwendung als Zwischenprodukte zur Synthese von Stoffen mit fungizider Wirksamkeit geht aus den folgenden Beispielen hervor.

Herstellungsbeispiele

Beispiel 1

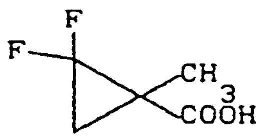

(I-1)

840 g (7,12 Mol) 2,2-Difluor-1-methyl-1-vinyl-cyclopropan in 10 l Wasser werden portionsweise mit 2,3 kg (14,47 Mol) Kaliumpermanganat versetzt. Man läßt 36 Stunden bei Raumtemperatur rühren, filtriert von Braunstein ab und wäscht gut mit Wasser nach. Das Filtrat wird mit konzentrierter Salzsäure sauer gestellt und mit Dichlormethan extrahiert. Nach dem Trocknen der organischen Phase wird das Lösungsmittel unter vermindertem Druck entfernt und der Rückstand destilliert.

Man erhält so 750 g (77 % der Theorie) 2,2-Difluor-1-methylcyclopropancarbonsäure vom Schmelzpunkt 59-61 °C.

Beispiel 2

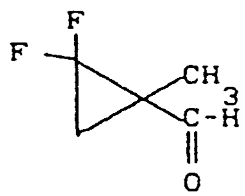

(I-2)

In eine Lösung von 30 g (0,25 Mol) 2,2-Difluor-1-methyl-1-vinyl-cyclopropan in 250 ml Methanol wird bei -78 °C unter Rühren Ozon eingeleitet. Danach tropft man eine Lösung von 50 g (0,4 Mol) Trimethylphosphit in 50 ml Methanol hinzu und läßt noch eine Stunde bei -78 °C weiterrühren. Anschließend wird das Reaktionsgemisch langsam auf Raumtempertur erwärmt und dann auf Wasser gegossen. Man extrahiert das entstehende Gemisch mehrfach mit Diethylether, trocknet die vereinigten organischen Phasen und destilliert unter vermindertem Druck. Auf diese Weise erhält man 9,2 g (31 % der Theorie) an 2,2-Difluor-1-methyl-cyclopropancarboxaldehyd in Form einer Flüssigkeit vom Siedepunkt 110-112 °C/200 Torr.

Beispiel 3

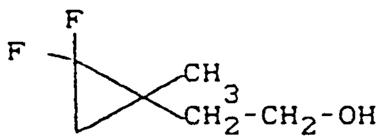

(I-3)

Aus 6,7 g (0,18 Mol) Natriumborhydrid und 33 g (0,23 Mol) Bortrifluorid-Etherat in 130 ml Diglyme werden 0,23 Mol Diboran erzeugt, die mit Hilfe eines Stickstoffstromes bei Raumtemperatur unter Rühren in eine Lösung von 30 g (0,25 Mol) 2,2-Difluor-1-methyl-1-vinyl-cyclopropan in 300 ml trockenem Diethylether eingeleitet werden. Das Reaktionsgemisch wird noch eine Stunde bei Raumtemperatur gerührt und durch Abziehen des Lösungsmittels und des überschüssigen Alkens unter vermindertem Druck eingeengt. Der verbleibende Rückstand wird in 50 ml Diethylether aufgenommen und mit 130 ml 10 %iger wäßriger Natronlauge versetzt. In dieses Gemisch werden bei Raumtemperatur unter Rühren 134 ml einer 30 %igen wäßrigen Wasserstoffperoxid-Lösung eingetropft. Man läßt noch eine Stunde bei Raumtemperatur rühren, extrahiert mehrfach mit Diethylether, trocknet die vereinigten organischen Phasen und destilliert unter vermindertem Druck. Auf diese Weise erhält man 13 g (55 % der Theorie) an 2-(2,2-Difluor-1-methyl-cyclopropyl)-ethanol in Form einer Flüssigkeit vom Siedepunkt 65-67 °C/20 Torr.

Beispiel 4

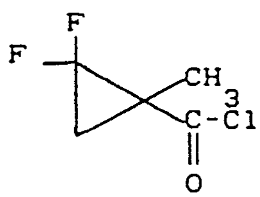

(I-4)

Ein Gemisch aus 250 g (1,8 Mol) 2,2-Difluor-1-methyl-cyclopropancarbonsäure und 700 ml Thionylchlorid wird in einer Destillationsapparatur unter Rühren langsam erhitzt, wobei zuerst überschüssiges Thionylchlorid und dann das gewünschte Produkt überdestilliert. Man erhält auf diese Weise 215 g (77 % der Theorie) an 2,2-Difluor-1-methyl-cyclopropan-carbonsäurechlorid in Form einer Flüssigkeit vom Siedepunkt 121-122 °C.

Beispiel 5

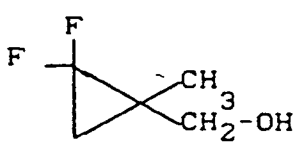

(I-5)

Zu einer Suspension von 25 g (0,66 Mol) Lithiumaluminiumhydrid in 250 ml trockenem Diethylether wird bei Raumtemperatur unter Rühren eine Lösung von 50 g (0,35 Mol) 2,2-Difluor-1-methyl-cyclopropan-carbonsäure in 50 ml Diethylether getropft. Anschließend erhitzt man 4 Stunden unter Rückfluß, läßt dann auf Raumtemperatur abkühlen und hydrolysiert vorsichtig durch langsame Zugabe von Wasser und konzentrierter Salzsäure. Man extrahiert das entstehende Gemisch mehrfach mit Diethylether, trocknet die vereinigten organischen Phasen und destilliert über eine VigreuxKolonne. Auf diese Weise erhält man 34,5 g (77 % der Theorie) an 2,2-Difluor-1-methyl-cyclopropyl-methanol in Form einer Flüssigkeit vom Siedepunkt 136-139 °C.

Beispiel 6

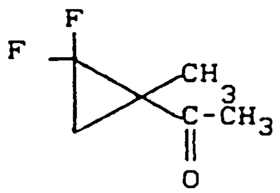

(I-6)

Zu 34 g (0,25 Mol) 2,2-Difluor-1-methylcyclopropancarbonsäure in 250 ml trockenem Diethylether tropft man unter Rühren bei -78 °C unter Stickstoff 333 ml einer 1,5-molaren Methyllithiumlösung (0,5 Mol). Man rührt noch eine Stunde bei -78 °C nach, erwärmt dann auf 0 °C und gießt die Reaktionslösung auf 500 g Eis und 50 ml konzentrierter Salzsäure. Die organische Phase wird abgetrennt und die wäßrige Phase mit Ether extrahiert. Die vereinigten organischen Phasen werden über Natriumsulfat getrocknet, das Lösungsmittel abdestilliert und das Produkt wird unter leicht vermindertem Druck destilliert.

Man erhält 21 g (63 % der Theorie) 2,2-Difluor-1-methylcyclopropylmethylketon vom Siedepunkt 58-60 °C/60 mbar.

Beispiel 7

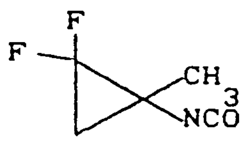

(I-7)

Zu einer Lösung von 28 g (0,24 Mol) Trimethylsilylazid in 120 ml Toluol werden bei 20 °C unter Rühren innerhalb von 10 Minuten 30,9 g (0,2 Mol) 2,2-Difluor-1-methyl-cyclopropan-carbonsäurechlorid hinzugetropft. Anschließend wird die Temperatur des Reaktionsgemisches unter Rühren innerhalb von 3 Stunden auf 90 °C gesteigert. Es wird so lange gerührt, bis die Gasentwicklung beendet ist. Der Fortgang der Reaktion wird IR-spektroskopisch verfolgt, indem durch Entnahme von Proben von Zeit zu Zeit die Abnahme der Azidbande bei 2130 $cm^{-1}$ ermittelt wird. Nach beendeter Umsetzung wird destilliert. Man erhält auf diese Weise 12,5 g (47 % der Theorie) an 2,2-Difluor-1-methyl-cyclopropyl-isocyanat in Form einer Flüssigkeit vom Siedepunkt 95-97 °C.

Beispiel 8

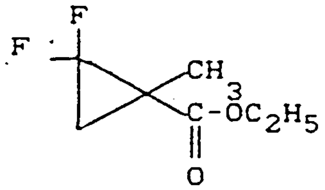

(I-8)

Unter Rühren bei 20°C werden 35 ml Ethanol zu 15,45 g (0,1 Mol) 2,2-Difluor-1-methyl-cyclopropan-carbonsäurechlorid getropft. Nach Zugabe von 2 Tropfen konzentrierter Schwefelsäure wird das Reaktionsgemisch noch eine Stunde unter Rückfluß erhitzt. Anschließend wird überschüssiges Ethanol abdestilliert, der Rückstand getrocknet und bei Normaldruck destilliert. Man erhält auf diese Weise 14,2 g (87 % der Theorie) an 2,2-Difluor-1-methyl-cyclopropan-carbonsäure-ethylester in Form einer Flüssigkeit vom Siedepunkt 141-143°C.

Beispiel 9

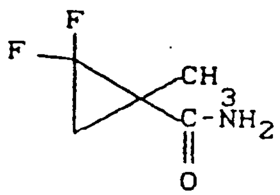

(I-9)

In eine Lösung von 15,45 g (0,1 Mol) 2,2-Difluor-1-methyl-cyclopropan-carbonsäurechlorid in 80 ml Dioxan wird 90 Minuten lang unter Rühren bei 20 bis 30°C Ammoniak-Gas eingeleitet. Anschließend wird die entstehende Suspension im Wasserstrahlvakuum bis zur Trockne eingeengt. Der verbleibende Rückstand wird mit 50 ml Wasser verrührt, abgesaugt und getrocknet. Man erhalt auf diese Weise 11 g (81 % der Theorie) an 2,2-Difluor-1-methyl-cyclopropan-carbonsäure-amid in Form einer Festsubstanz vom Schmelzpunkt 125-127°C.

Beispiel 10

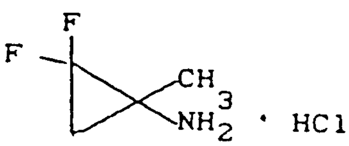

(I-10)

Eine Lösung von 13 g (0,1 Mol) 2,2-Difluor-1-methyl-cyclopropyl-isocyanat in 100 ml Toluol wird mit 50 ml konzentrierter Salzsäure versetzt und 16 Stunden bei Raumtemperatur gerührt. Anschließend erhitzt man das Reaktionsgemisch noch eine Stunde auf 50°C und zieht dann das Lösungsmittel unter vermindertem Druck vollständig ab. Es verbleiben 9 g (63 % der Theorie) an 2,2-Difluor-1-methyl-cyclopropylamin-hydrochlorid in Form einer Festsubstanz vom Schmelzpunkt 140-142°C (Zersetzung).

Beispiel 11

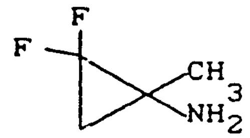

(I-11)

In ein Gemisch aus 7,2 g (0,05 Mol) 2,2-Difluor-1-methyl-cyclopropylamin-hydrochlorid und 20 ml Wasser werden bei 0 bis 5 ° C unter Rühren 12 g (0,06 Mol) 20 %ige wäßrige Natronlauge getropft. Das Reaktionsgemisch wird zweimal mit Diethylether extrahiert, die vereinigten organischen Phasen werden über Magnesiumsulfat getrocknet und unter vermindertem Druck eingeengt. Der verbleibende Rückstand wird unter vermindertem Druck destilliert. Man erhält auf diese Weise 3,9 g (73 % der Theorie) an 2,2-Difluor-1-methyl-cyclopropylamin in Form einer Flüssigkeit vom Siedepunkt 25-30 ° C/15 mbar.

Herstellung von Verbindungen der Formeln (V) bzw. (XVII):

Beispiel 12

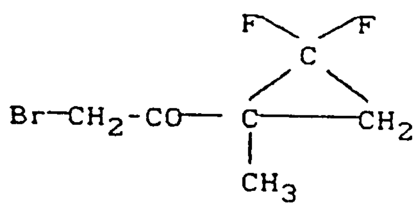

(VI-1)

Zur Lösung von 12,5 g (0,09 Mol) 1-Acetyl-1-methyl-2,2-difluor-cyclopropan in 50 ml Methanol tropft man unter Rühren bei 20 ° C eine Lösung von 4,7 ml (0,09 Mol) Brom in 30 ml Methylenchlorid. Nach vollständiger Entfärbung gießt man das Reaktionsgemisch in 200 ml Wasser und extrahiert mit Methylenchlorid. Nach dem Einengen unter vermindertem Druck erhält man 20 g (GC-Gehalt 47 %; 47 % der Theorie) rohes 1-Bromacetyl-1-methyl-2,2-difluorcyclopropan,das ohne weitere Reinigung direkt umgesetzt wird.

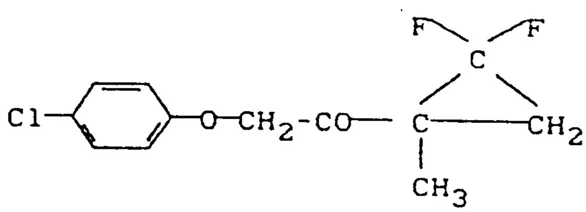

(VIII-1)

6 g (0,028 Mol) 1-Bromacetyl-1-methyl-2,2-difluorcyclopropan werden in 30 ml Aceton unter Zusatz von 4,4 g (0,032 Mol) Kaliumcarbonat und 4,1 g (0,032 Mol) 4-Chlorphenol 16 Stunden unter Rühren unter Rückfluß erhitzt. Man verdünnt mit Wasser, extrahiert das Produkt mit Methylenchlorid, wäscht die organische Phase einmal mit verdünnter Natronlauge, dann mit Wasser und engt unter vermindertem Druck ein.

Man erhält 6 g 1-(4-Chlorphenoxyacetyl)-1-methyl-2,2-difluorcyclopropan als Rohprodukt mit einem Gehalt (GC) von 66 % (54,4 % der Theorie).

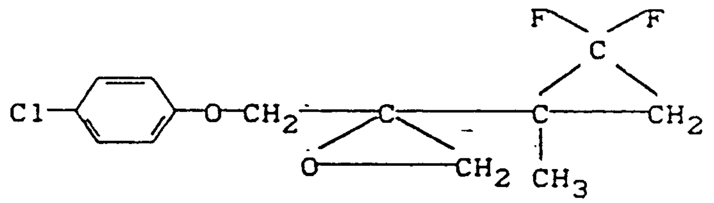

(XI-1)

Zur Losung von 30 ml (0,04 Mol) einer 1,36 molaren Lösung von Trimethylsulfonium-methylsulfat in Acetonitril fügt man 2,4 g (0,044 Mol) Natriummethylat hinzu und rührt 30 Minuten bei Raumtemperatur. Dann setzt man 5,8 g (0,022 Mol) 1-(4-Chlorphenoxyacetyl)-1-methyl-2,2-difluorcyclopropan zu und läßt 16 Stunden bei 20 ° C rühren. Man gießt das Reaktionsgemisch in 200 ml Wasser, extrahiert mit Methylenchlorid, wäscht die organische Phase mit Wasser und engt die organische Phase ein.

Man erhält 4,3 g (GC-Gehalt von 73 %; 71,6 % der Theorie) 2-(4-Chlorphenoxymethyl)-2-(2,2-difluor-1-methyl-cyclopropyl)-oxiran als zähes Harz, das direkt weiter umgesetzt wird.

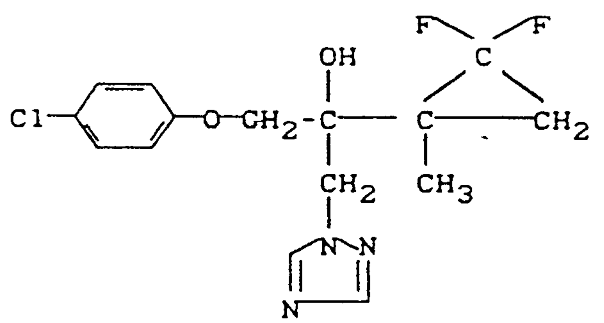

(V-1)

4,2 g (0,015 Mol) 2-(4-Chlorphenoxymethyl)-2-(2,2-difluor-1-methylcyclopropyl)-oxiran, 2,1 g (0,03 Mol) 1,2,4-Triazol und 4,2 g (0,03 Mol) Kaliumcarbonat werden in 30 ml Dimethylformamid 16 Stunden bei 90 ° C gerührt.

Nach dem Entfernen des Lösungsmittels an der Ölpumpe wird der Rückstand mit Wasser/Methylenchlorid verrührt, die organische Phase abgetrennt und eingeengt. Der Rückstand wird mittels Säulenchromatographie (Chloroform/Essigester = 4:1) an Kieselgel gereinigt.

Man erhält 2,3 g (44,6 % der Theorie) 1-(4-Chlorphenoxy)-2-(2,2-difluor-1-methylcyclopropyl)-2-hydroxy-3-(1,2,4-triazol-1-yl)-propan vom Schmelzpunkt 110 ° C.

Beispiel 13

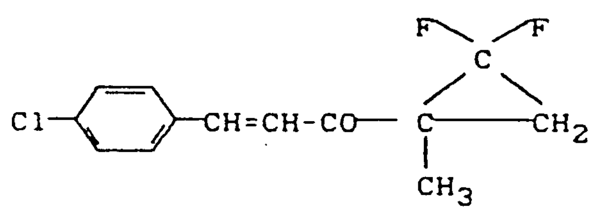

(XIV-1)

15 g (0,11 Mol 1-Acetyl-2,2-difluor-1-methylcyclopropan in 20 ml 10%-iger methanolischer Natronlauge werden unter Rühren mit einer Lösung von 15,5 g (0,11 Mol) p-Chlorbenzaldehyd in 20 ml Methanol bei 20 ° C versetzt. Nach 15 Minuten fügt man weitere 40 ml 10 %-ige methanolische Natronlauge zu und rührt weitere 16 Stunden bei Raumtemperatur. Man gießt das Reaktionsgemisch in Wasser, extrahiert mit Methylenchlorid, wäscht die organische Phase mit Wasser und engt unter vermindertem Druck ein.

Man erhält 25 g (88 % der Theorie) rohes (2,2-Difluor-1-methylcyclopropyl)-[2-(4-chlorphenyl)-ethen-1-yl]-keton als Öl, das direkt weiter umgesetzt wird.

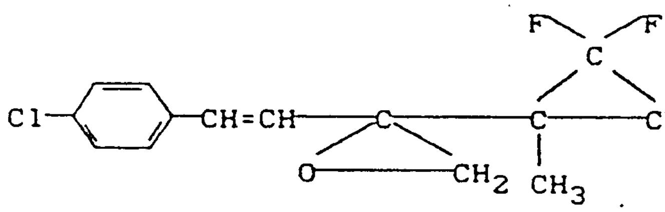

(XV-1)

41 ml (0,06 Mol) einer 1,46-molaren Lösung von Trimethylsulfoniummethylsulfat in Acetonitril werden mit 3,6 g (0,066 Mol) Natriummethylat versetzt und 30 Minuten bei Raumtemperatur gerührt. Anschließend fügt man eine Lösung von 10 g (0,039 Mol) (2,2-Difluor-1-methyl-cyclopropyl)-[ 2-(4-chlorphenyl)-ethen-1-yl]-keton in 100 ml Acetonitril hinzu und rührt 16 Stunden bei Raumtemperatur. Man gießt dann das Reaktionsgemisch in Wasser, extrahiert mit Methylenchlorid, wäscht die organische Phase zweimal mit Wasser und engt die organische Phase unter vermindertem Druck ein.

Man erhält 6,8 g (GC-Gehalt 38 %; 24 % der Theorie) 2-(2,2-Difluor-1-methylcyclopropyl)-2-[2-(4-chlorphenyl)-ethen-1-yl]-oxiran, das ohne weitere Reinigung direkt umgesetzt wird.

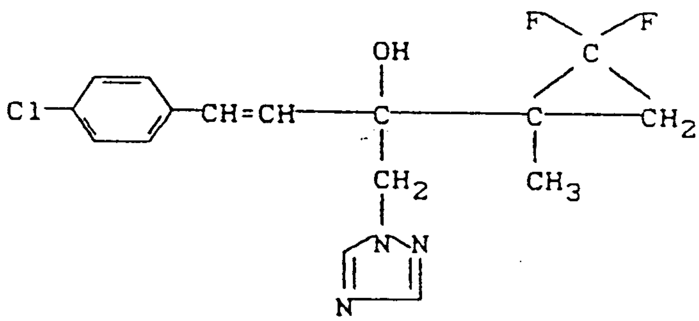

(V-2)

Zur Lösung von 3,3 g (0,048 Mol) 1,2,4-Triazol in 30 ml Dimethylformamid fügt man portionsweise 1,4 g (0,048 Mol) 80 %iges Natriumhydrid und rührt 30 Minuten bei 20-30 °C. Anschließend setzt man 6,6 g (0,024 Mol) 2-(2,2-Difluor-1-methyl-cyclopropyl)-2-[2-(4-chlorphenyl)-ethen-1-yl]-oxiran zu und läßt 16 Stunden bei 90 °C rühren. Man gießt das Reaktionsgemisch dann auf Wasser, extrahiert mit Methylenchlorid, engt die organische Phase unter vermindertem Druck ein und reinigt den Rückstand durch Chromatographie an Kieselgel (Chloroform/Essigester = 4:1).

Man erhalt 2,4 g (29,6 % der Theorie) 1-(4-Chlorphenyl)-3-(2,2-difluor-1-methylcyclopropyl)-3-hydroxy-4-(1,2,4-triazol-1-yl)-but-1-en vom Schmelzpunkt 131 °C.

Nach dem in den Beispielen 12 und 13 angegebenen Methoden sowie gemäß den in der Beschreibung offenbarten Verfahren werden auch die in der folgenden Tabelle aufgeführten Stoffe der Formel (V) erhalten.

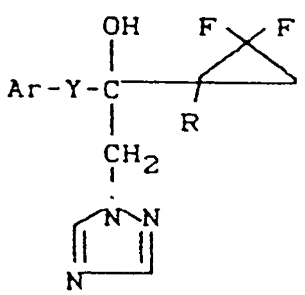

(V)

| Beispiel-Nr. | Verbindungs-Nr. | Ar | Y | R | physikal. Konstante Fp(°C) |
|---|---|---|---|---|---|
| 14 | V-3 | 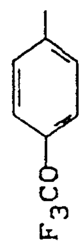 | $-OCH_2-$ | $CH_3$ | Harz |
| 15 | V-4 | 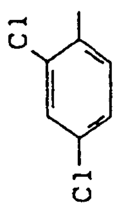 | $-OCH_2-$ | $CH_3$ | Harz |
| 16 | V-5 | 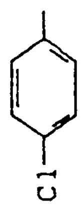 | $-CH_2CH_2-$ | $CH_3$ | 112 |
| 17 | V-6 | 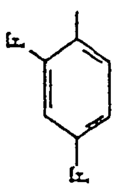 | $-OCH_2-$ | $CH_3$ | Harz |
| 18 | V-7 | 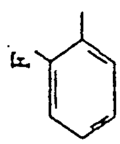 | $-OCH_2-$ | $CH_3$ | Harz |
| 19 | V-8 | 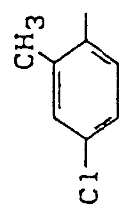 | $-OCH_2-$ | $CH_3$ | Harz |

Beispiel 20

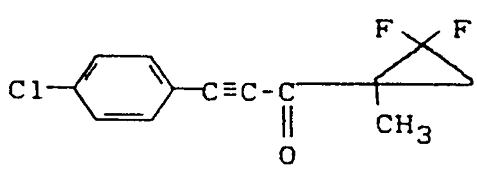

(XIX-1)

Ein Gemisch aus 10,1 g (0,1 Mol) Triethylamin, 1,4 g (0,01 Mol) Kupfer-(I)-bromid und 55 ml Toluol wird mit 13,7 g (0,1 Mol) p-Chlorphenyl-acetylen versetzt und 30 Minuten bei Raumtemperatur unter Argon gerührt. Nach dem Erhitzen des Reaktionsgemisches auf 55 °C werden 15,5 g (0,1 Mol) 1-Methyl-2,2-difluor-cyclopropancarbonsäurechlorid zugetropft. Danach wird 8 Stunden bei 90 °C gerührt, dann abgekühlt und filtriert.Das Filtrat wird nacheinander mit verdünnter, wäßriger Salzsäure und Wasser gewaschen und unter vermindertem Druck eingeengt. Es verbleiben 19,2 g eines dunklen Öles, das im Kugelrohr destilliert

wird. Bei 0,1 mbar und einer Manteltemperatur von 120 ° C geht ein gelbes Öl über, das beim Stehen erstarrt. Man erhält 8,4 g (33 % der Theorie) an [2-(4-Chlorphenyl)-ethin-1-yl]-(2,2-difluor-1-methyl-cycloprop-1-yl)-keton vom Schmelzpunkt 67-68 ° C.

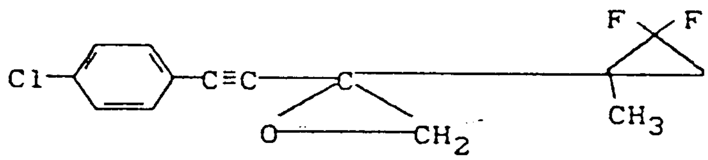

(XX-1)

Ein Gemisch aus 13 g Dimethylsulfid und 4,8 g Dimethylsulfat wird zunächst 2 Stunden bei Raumtemperatur gerührt und dann mit 18 ml tert.-Butanol sowie mit 8,9 g [2-(4-Chlorphenyl)-ethin-1-yl]-(2,2-difluor-1-methyl-cycloprop-1-yl)-keton versetzte Man rührt 30 Minuten bei Raumtemperatur, kühlt auf 10 ° C ab und tropft unter Rühren innerhalb von einer Stunde eine Losung von 4,5 g Kalium-tert.-butylat in 32 ml tert.-Butanol hinzu. Das Reaktionsgemisch wird noch 3 Stunden bei 10 ° C nachgerührt und dann durch Abziehen des Verdünnungsmittels unter vermindertem Druck eingeengt.Man nimmt den Rückstand in Methylenchlorid auf, wascht dreimal mit Wasser, trocknet und engt erneut unter vermindertem Druck ein. Es verbleiben 6,4 g (68 % der Theorie) an 2-[2-(4-Chlorphenyl)-ethin-1-yl]-2-(2,2-difluor-1-methyl-cycloprop-1-yl)-oxiran in Form eines Öles, das ohne zusätzliche Reinigung weiter umgesetzt wird.

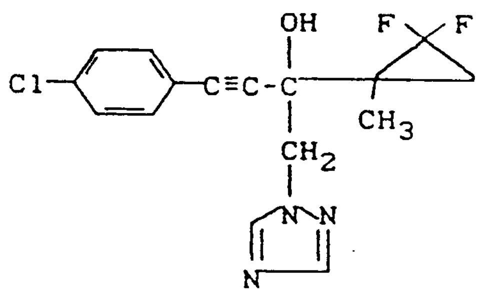

(XVII-1)

In ein Gemisch aus 0,9 g (0,013 Mol) 1,2,4-Triazol, 1,8 g (0,013 Mol) gemahlenem Kaliumcarbonat und 30 ml Acetonitril werden bei Raumtemperatur unter Rühren 3,5 g 2-[2-(4-Chlorphenyl)-ethin-1-yl]-2-(2,2-difluor-1-methyl-cycloprop-1-yl)-oxiran gegeben. Das Gemisch wird 8 Stunden unter Rückfluß erhitzt, dann abgekühlt, mit Wasser verdünnt und dreimal mit Methylenchlorid extrahiert. Die vereinigten organischen Phasen werden mit Wasser gewaschen, dann getrocknet und unter vermindertem Druck eingeengt. Man erhält auf diese Weise 3,8 g eines dunklen Öles, das durch Säulenchromatographie (Petrolether/Essigester = 2:1) an Kieselgel gereinigt wird. Es verbleiben 2,1 g (48 % der Theorie) an 1-(4-Chlorphenyl)-3-(2,2-difluor-1-methyl-cycloprop-1-yl)-4-(1,2,4-triazol-1-yl)-but-1-in-3-ol in Form einer Festsubstanz vom Schmelzpunkt 113-115 ° C.

In den folgenden Verwendungsbeispielen wurden die Verbindungen der nachstehend angegebenen Formeln als Vergleichssubstanzen eingesetzt:

(A) =

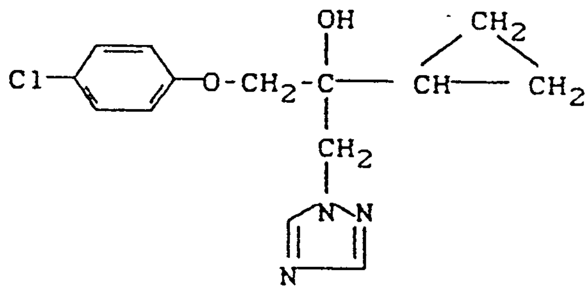

(Bekannt aus EP-A 0 040 345)

(B) = 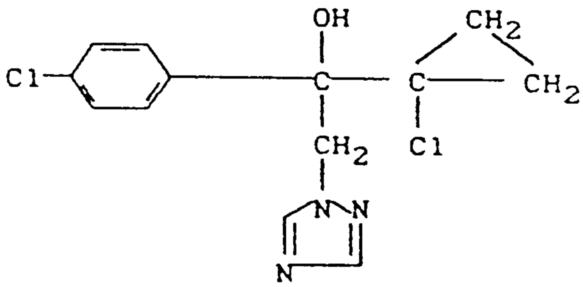

(Bekannt aus EP-A 0 180 136)

(C) = 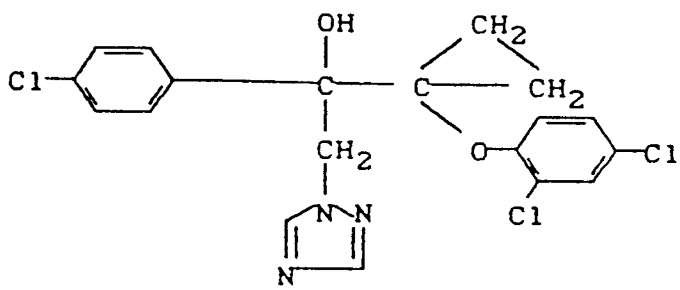

(Bekannt aus EP-A 0 180 136)

(D) = 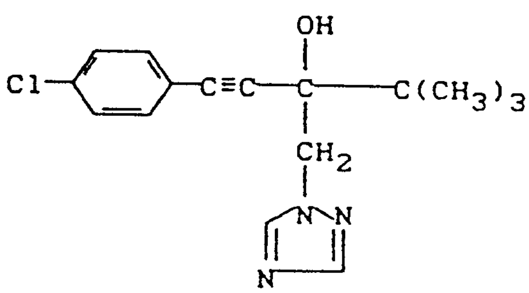

(Bekannt aus EP-A 0 052 424)

Beispiel A

Erysiphe-Test (Weizen) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.tritici bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die Verbindungen (V-1), (V-2), (V-3) und (V-4) eine wesentlich bessere Wirksamkeit als die Vergleichssubstanzen (B) und (C).

Beispiel B

Puccinia-Test (Weizen) / protektiv

Lösungsmittel: 100 Gewichtsteile Dimethylformamid

Emulgator: 0,025 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert Nach Antrocknen besprüht man die Pflanzen mit der Wirkstoffzubereitung taufeucht. Die Pflanzen verbleiben 24 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt; um die Entwicklung von Rostpusteln zu begünstigen.

10 Tage nach der Inokulation erfolgt die Auswertung.

In diesem Test zeigen die Verbindungen (V-1), (V-2), (V-3), (V-4) und (V-5) eine wesentlich bessere Wirksamkeit als die Vergleichssubstanz (A).

Beispiel C

Venturia-Test (Apfel) /protektiv

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

In diesem Test zeigen die Verbindungen (V-1), (V-2) und (V-5) eine wesentlich bessere Wirksamkeit als die Vergleichssubstanz (C).

Beispiel D

Erysiphe-Test (Gerate) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die Verbindung (XVII-1) eine wesentlich bessere Wirkung als die Vergleichssubstanz (D).

Beispiel E

Uromyces-Test (Buschbohne) / protektiv

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die

gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Uredosporensuspension des Bohnenrosterregers (Uromyces appendiculatus) inokuliert und verbleiben 1 Tag in einer dunklen Feuchtkammer bei 20 bis 22° C und 100 % relativer Luftfeuchtigkeit.

Die Pflanzen werden dann unter intensiver Belichtung für 9 Tage bei 20 bis 22° C und einer relativen Luftfeuchtigkeit von 70 bis 80 % im Gewächshaus aufgestellt.

10 Tage nach der Inokulation erfolgt die Auswertung.

In diesem Test zeigt die Verbindung (XVII-1) eine bessere Wirksamkeit als die Vergleichssubstanz (D).

Beispiel F

Pyricularia-Test (Reis) /protektiv

Lösungsmittel: 12,5 Gewichtsteile Aceton

Emulgator: 0,3 Gewichtsteile Alkylarylpolyglkyolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25° C aufgestellt.

4 Tage nach der Inokulation erfolgt die Auswertung des Krankheitsbefalles.

In diesem Test zeigt die Verbindung (XVII-1) eine wesentlich bessere Wirksmkeit als die Vergleichssubstanz (D).

**Patentansprüche**

**1.** 2,2-Difluorcyclopropyl-Derivate der Formel

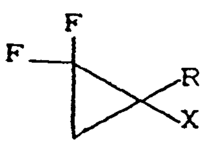

(I)

in welcher

R für Alkyl mit 1 bis 4 Kohlenstoffatomen steht oder

für Benzyl steht, das im Phenylteil einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Alkoximinoalkyl mit 1 bis 4 Kohlenstoffatomen im Alkylteil und 1 bis 4 Kohlenstoffatomen im Alkoxyteil, gegebenenfalls durch Alkyl mit 1 oder 2 Kohlenstoffatomen und/oder Halogen substituiertes Phenoximinoalkyl mit 1 bis 4 Kohlenstoffatomen im Alkylteil, gegebenenfalls durch Alkyl mit 1 oder 2 Kohlenstoffatomen und/oder Halgoen substituiertes Phenyl oder gegebenenfalls durch Alkyl mit 1 oder 2 Kohlenstoffatomen und/oder Halogen substituiertes Phenoxy,

X für Hydroxymethyl, 2-Hydroxyethyl, Isocyanato, Amine oder Amino-hydrochlorid steht oder für den Rest der Formel -CO-$R^1$ steht, worin

$R^1$ für Wasserstoff, Hydroxy, Alkoxy mit 1 bis 4 Kohlenstoffatomen, Alkyl mit 1 bis 4 Kohlenstoffatomen, Chlor, oder Amino steht.

**2.** Verfahren zur Herstellung von 2,2-Difluorcyclopropyl-Derivaten der Formel (I) gemäß Anspruch 1,

dadurch gekennzeichnet, daß man

a) Vinylcyclopropan-Derivate der Formel

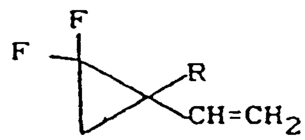

(II)

in welcher

R die oben angegebene Bedeutung hat,

entweder

α) mit einem starken Oxidationsmittel in Gegenwart eines Verdünnungsmittels umsetzt,

oder

β) mit Ozon in Gegenwart eines Verdünnungsmittels sowie mit einem Reduktionsmittel umsetzt,

oder

γ) zunächst in einer ersten Stufe mit Diboran in Gegenwart eines Verdünnungsmittels umsetzt und das dabei entstehende Produkt danach in einer zweiten Stufe mit einem starken Oxidationsmittel in Gegenwart eines Verdünnungsmittels umsetzt,

oder

b) 2,2-Difluorcyclopropyl-Derivate der Formel

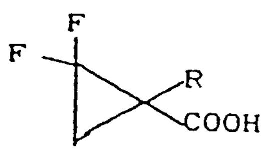

(Ia)

in welcher

R die oben angegebene Bedeutung hat

entweder

α) mit Thionylchlorid, Sulfurylchlorid oder Phosphortrichlorid gegebenenfalls in Gegenwart eines Verdünnungsmittels umsetzt,

oder

β) mit Lithium-aluminium-hydrid in Gegenwart eines Verdünnungsmittels umsetzt,

oder

γ) mit metallorganischen Verbindungen der Formel

$R^2$-Li (III)

in welcher

$R^2$ für Alkyl mit 1 bis 6 Kohlenstoffatomen steht,

in Gegenwart eines Verdünnungsmittels umsetzt.

oder

c) 2,2-Difluorcyclopropyl-Derivate der Formel

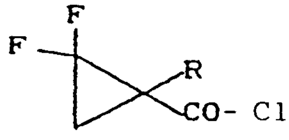

(Ib)

in welcher

R die oben angegebene Bedeutung hat ,

entweder

α) mit einem Azidgruppen übertragenden Reagenz in Gegenwart eines Verdünnungsmittels umsetzt und die dabei entstehenden Verbindungen der Formel

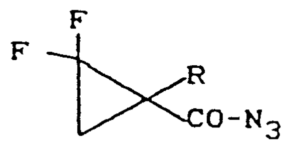

(Ic)

in welcher

R die oben angegebene Bedeutung hat,

in Gegenwart eines Verdünnungsmittels thermisch zersetzt,

oder

β) mit Alkoholen der Formel

$R^3$-OH (IV)

in welcher

$R^3$ für Alkyl mit 1 bis 6 Kohlenstoffstomen steht,

gegebenenfalls in Gegenwart eines Katalysators sowie gegebenenfalls in Gegenwart eines Verdünnungsmittels umsetzt,

oder

γ) mit Ammoniak gegebenenfalls in Gegenwart eines Verdünnungsmittels umsetzt,

oder

d) 2,2-Difluorcyclopropyl-Derivate der Formel

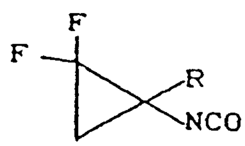

(Id)

in welcher

R die oben angegebene Bedeutung hat

mit Chlorwasserstoff-Säure in Gegenwart eines Verdünnungsmittels umsetzt und gegebenenfalls die dabei entstehenden Verbindungen der Formel

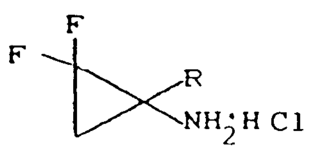

(Ie)

in welcher

R die oben angegebene Bedeutung hat und

mit Basen gegebenenfalls in Gegenwart eines Verdünnungsmittels umsetzt.

## Claims

**1.** 2,2-Difluorocyclopropyl derivatives of the formula

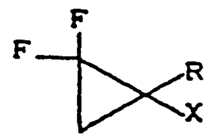

(I)

in which

R represents alkyl having 1 to 4 carbon atoms or benzyl which can be monosubstituted to trisubstituted in the phenyl moiety by identical or different substituents from the series comprising halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, alkoximinoalkyl having 1 to 4 carbon atoms in the alkyl moiety and 1 to 4 carbon atoms in the alkoxy moiety, phenoximinoalkyl which has 1 to 4 carbon atoms in the alkyl moiety and which is optionally substituted by alkyl having 1 or 2 carbon atoms and/or halogen, phenyl which is optionally substituted by alkyl having 1 or 2 carbon atoms and/or halogen, or phenoxy which is optionally substituted by alkyl having 1 or 2 carbon atoms and/or halogen, and

X represents hydroxymethyl, 2-hydroxyethyl, isocyanato, amino or amino hydrochloride, or represents the radical of the formula $-CO-R^1$, where

$R^1$ represents hydrogen, hydroxyl, alkoxy having 1 to 4 carbon atoms, alkyl having 1 to 4 carbon atoms, chlorine or amino.

**2.** Process for the preparation of 2,2-difluorocyclopropyl derivatives of the formula (I) according to Claim 1, characterised in that

a) vinylcyclopropane derivatives of the formula

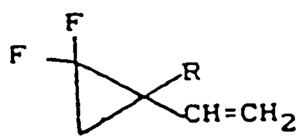

(II)

in which

R has the abovementioned meaning,

are reacted either

α) with a strong oxidant in the presence of a diluent,

or

β) with ozone in the presence of a diluent and with a reducing agent,

or

γ) initially in a first step with diborane in the presence of a diluent and the resulting product is then reacted in a second step with a strong oxidant in the presence of a diluent,

or

b) 2,2-difluorocyclopropyl derivatives of the formula

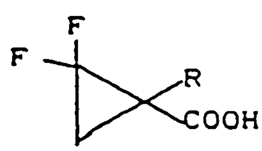

(Ia)

in which

R has the abovementioned meaning, are reacted either

α) with thionyl chloride, sulphuryl chloride or phosphorus trichloride, if appropriate in the

presence of a diluent,

or

β) with lithium aluminium hydride in the presence of a diluent,

or

γ) with organometallic compounds of the formula

$R^2$-Li (III)

in which

$R^2$ represents alkyl having 1 to 6 carbon atoms, in the presence of a diluent,

or

c) 2,2-difluorocyclopropyl derivatives of the formula

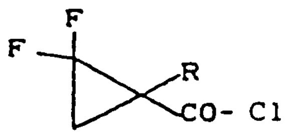
(Ib)

in which

R has the abovementioned meaning,

are reacted either

α) with an azide group-transferring reagent in the presence of a diluent and the resultant compounds of the formula

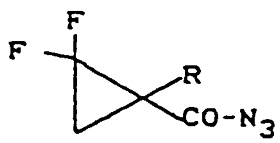
(Ic)

in which

R has the abovementioned meaning,

are thermally decomposed in the present of a diluent, or

β) with alcohols of the formula

$R^3$-OH (IV)

in which

$R^3$ represents alkyl having 1 to 6 carbon atoms,

if appropriate in the present of a catalyst and if appropriate in the presence of a diluent,

or

γ) with ammonia, if appropriate in the presence of a diluent,

or

d) 2,2-difluorocyclopropyl derivatives of the formula

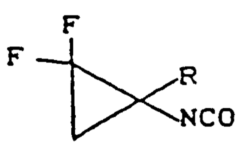
(Id)

in which

R has the abovementioned meaning,

are reacted with hydrochloric acid in the presence of a diluent and if appropriate the resultant

compounds of the formula

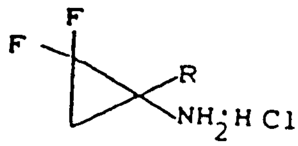

(Ie)

in which

R has the abovementioned meaning,

are reacted with bases, if appropriate in the presence of a diluent.

**Revendications**

1. Dérivés 2,2-difluorocyclopropyliques de formule

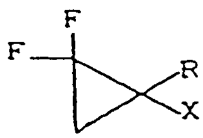

(I)

dans laquelle

R est un groupe alkyle ayant 1 à 4 atomes de carbone ou un groupe benzyle qui peut porter dans la partie phényle 1 à 3 substituants, identiques ou différents, halogéno, alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, alkoximinoalkyle ayant 1 à 4 atomes de carbone dans la partie alkyle et 1 à 4 atomes de carbone dans la partie alkoxy, phénoximinoalkyle ayant 1 à 4 atomes de carbone dans la partie alkyle, éventuellement substitué par un radical alkyle ayant 1 ou 2 atomes de carbone et/ou par un halogène, phényle éventuellement substitué par un radical alkyle ayant 1 ou 2 atomes de carbone et/ou par un halogène ou phénoxy éventuellement substitué par un radical alkyle ayant 1 ou 2 atomes de carbone et/ou par un halogène,

X est un groupe hydroxyméthyle, 2-hydroxyéthyle, isocyanato, amino ou chlorhydrate d'amine, ou le reste de formule $-CO-R^1$ dans laquelle

$R^1$ est l'hydrogène, un groupe hydroxy, alkoxy ayant 1 à 4 atomes de carbone, alkyle ayant 1 à 4 atomes de carbone, du chlore ou un groupe amino.

2. Procédé de production de dérivés 2,2-difluorocyclopropyliques de formule (I) suivant la revendication 1, caractérisé en ce qu'on fait réagir

a) des dérivés de vinylcyclopropane de formule

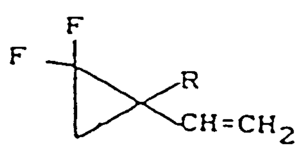

(II)

dans laquelle

R a la définition indiquée ci-dessus,

ou bien

α) avec un oxydant fort en présence d'un diluant,

ou bien

β) avec l'ozone en présence d'un diluant ainsi qu'avec un agent réducteur,

ou bien

γ) tout d'abord dans une première étape avec du diborane en présence d'un diluant, puis on fait réagir le produit obtenu dans une seconde étape avec un agent oxydant fort en présence d'un diluant,

ou bien

b) des dérivés 2,2-difluorocyclopropyliques de formule

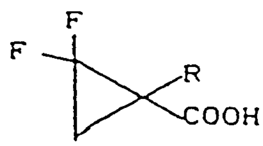

(Ia)

dans laquelle

R a la définition indiquée ci-dessus, ou bien

α) avec le chlorure de thionyle, le chlorure de sulfuryle ou le trichlorure de phosphore éventuellement en présence d'un diluant,

ou bien

β) avec l'hydrure de lithium et d'aluminium en présence d'un diluant,

ou bien

γ) avec des composés organométalliques de formule

$R^2$-Li (III)

dans laquelle

$R^2$ est un groupe alkyle ayant 1 à 6 atomes de carbone,

en présence d'un diluant,

ou bien

c) des dérivés 2,2-difluorocyclopropyliques de formule

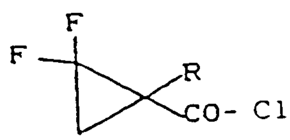

(Ib)

dans laquelle

R a la définition indiquée ci-dessus,

ou bien

α) avec un réactif transférant des groupes azide en présence d'un diluant, et on décompose thermiquement les composés qui sont alors produits, de formule

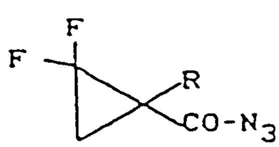

(Ic)

dans laquelle

R a la définition indiquée ci-dessus,

en présence d'un diluant, ou bien

β) avec des alcools de formule

$R^3$-OH (IV)

dans laquelle

$R^3$ est un groupe alkyle ayant 1 à 6 atomes de carbone,

éventuellement en présence d'un catalyseur ainsi que, le cas échéant, en présence d'un diluant,

ou bien

γ) avec l'ammoniac, le cas échéant en présence d'un diluant,

ou bien

d) des dérivés 2,2-difluorocyclopropyliques de formule

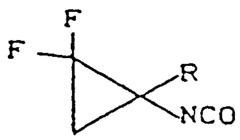

(Id)

dans laquelle

R a la définition indiquée ci-dessus avec l'acide chlorhydrique en présence d'un diluant et on fait réagir lé cas échéant les composés qui sont alors produits, de formule

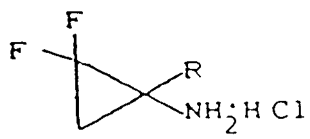

(Ie)

dans laquelle

R a la définition indiquée ci-dessus

avec des bases, éventuellement en présence d'un diluant.